# EUROPEAN PATENT APPLICATION

(11) **EP 2 327 428 A2**
(43) Date of publication of application: **01.06.2011**
(21) Application number: 10191742.5
(22) Date of filing: 18.11.2010
(51) Int. Cl.: A61L 27/22, A61L 27/38, A61L 27/50

(54) **Pharmaceutical compositions for repairing or replacing damaged tissues, and for improving myocardial infarction**

(30) Priority: 18.11.2009 US 620890
(71) Applicant: National Cheng Kung University, Tainan City 701 (TW)
(72) Inventor: HSIEH, Ching-Ho, Tainan City 701 (TW); LIN, Yi-Dong, Tainan City 701 (TW); YANG, Yu-Jen, Tainan City 701 (TW); YEH, Ming-Long, Tainan City 701 (TW)
(74) Representative: Rowe, Daniel

(57) **Abstract**

The invention provides a pharmaceutical composition for repairing, or replacing a damaged tissue, and a pharmaceutical composition for improving myocardial infarction. The pharmaceutical compositions of the present invention comprise: a biologically compatible peptide hydrogel comprising at least one self-assembling peptide nanofiber, wherein the self-assembling peptide nanofibers are complementary to each other; and at least one cell selected from the group consisting of autologous stem cells, progenitor cells, committed cells, and mature somatic cells.

SEQUENCE LISTING

<110> National Cheng Kung University

<120> Pharmaceutical composition for repairing or replacing a damaged
tissue, or improving myocardial infraction

<130> 463.107768

<150> US 12/620,890
<151> 2009-11-18

<160> 51

<170> PatentIn version 3.3

<210> 1
<211> 16
<212> PRT
<213> Artificial

<220>
<223> Synthetic self-assembling peptide

<400> 1

Ala Lys Ala Lys Ala Glu Ala Glu Ala Lys Ala Lys Ala Glu Ala Glu
1               5                   10                  15

<210> 2
<211> 16
<212> PRT
<213> Artificial

<220>
<223> Synthetic self-assembling peptide

<400> 2

Ala Lys Ala Glu Ala Lys Ala Glu Ala Lys Ala Glu Ala Lys Ala Glu
1               5                   10                  15

<210> 3
<211> 16
<212> PRT
<213> Artificial

<220>
<223> Synthetic self-assembling peptide

<400> 3

Glu Ala Lys Ala Glu Ala Lys Ala Glu Ala Lys Ala Glu Ala Lys Ala
1               5                   10                  15

<210> 4
<211> 16
<212> PRT
<213> Artificial

<220>
<223> Synthetic self-assembling peptide

<400> 4

Lys Ala Glu Ala Lys Ala Glu Ala Lys Ala Glu Ala Lys Ala Glu Ala
1               5                   10                  15

<210>  5
<211>  16
<212>  PRT
<213>  Artificial

<220>
<223>  Synthetic self-assembling peptide

<400>  5

Ala Glu Ala Lys Ala Glu Ala Lys Ala Glu Ala Lys Ala Glu Ala Lys
1               5                   10                  15

<210>  6
<211>  16
<212>  PRT
<213>  Artificial

<220>
<223>  Synthetic self-assembling peptide

<400>  6

Ala Asp Ala Asp Ala Arg Ala Arg Ala Asp Ala Asp Ala Arg Ala Arg
1               5                   10                  15

<210>  7
<211>  16
<212>  PRT
<213>  Artificial

<220>
<223>  Synthetic self-assembling peptide

<400>  7

Ala Arg Ala Asp Ala Arg Ala Asp Ala Arg Ala Asp Ala Arg Ala Asp
1               5                   10                  15

<210>  8
<211>  16
<212>  PRT
<213>  Artificial

<220>
<223>  Synthetic self-assembling peptide

<400>  8

Asp Ala Arg Ala Asp Ala Arg Ala Asp Ala Arg Ala Asp Ala Arg Ala
1               5                   10                  15

<210>  9
<211>  16
<212>  PRT
<213>  Artificial

<220>
<223>  Synthetic self-assembling peptide

<400>  9

Arg Ala Asp Ala Arg Ala Asp Ala Arg Ala Asp Ala Arg Ala Asp Ala
1               5                   10                  15


<210>  10
<211>  16
<212>  PRT
<213>  Artificial

<220>
<223>  Synthetic self-assembling peptide

<400>  10

Ala Asp Ala Arg Ala Asp Ala Arg Ala Asp Ala Arg Ala Asp Ala Arg
1               5                   10                  15


<210>  11
<211>  16
<212>  PRT
<213>  Artificial

<220>
<223>  Synthetic self-assembling peptide

<400>  11

Ala Arg Ala Asp Ala Lys Ala Glu Ala Arg Ala Asp Ala Lys Ala Glu
1               5                   10                  15


<210>  12
<211>  16
<212>  PRT
<213>  Artificial

<220>
<223>  Synthetic self-assembling peptide

<400>  12

Ala Lys Ala Glu Ala Arg Ala Asp Ala Lys Ala Lys Ala Arg Ala Asp
1               5                   10                  15


<210>  13
<211>  16
<212>  PRT
<213>  Artificial

<220>
<223>  Synthetic self-assembling peptide

<400>  13

Ala Arg Ala Lys Ala Asp Ala Glu Ala Arg Ala Lys Ala Asp Ala Glu
1               5                   10                  15


<210>  14
<211>  16
<212>  PRT
<213>  Artificial

<220>

<223>  Synthetic self-assembling peptide

<400>  14

Ala Lys Ala Arg Ala Glu Ala Asp Ala Lys Ala Arg Ala Glu Ala Asp
1               5                   10                  15


<210>  15
<211>  16
<212>  PRT
<213>  Artificial

<220>
<223>  Synthetic self-assembling peptide

<400>  15

Ala Gln Ala Gln Ala Gln Ala Gln Ala Gln Ala Gln Ala Gln Ala Gln
1               5                   10                  15


<210>  16
<211>  16
<212>  PRT
<213>  Artificial

<220>
<223>  Synthetic self-assembling peptide

<400>  16

Val Gln Val Gln Val Gln Val Gln Val Gln Val Gln Val Gln Val Gln
1               5                   10                  15


<210>  17
<211>  16
<212>  PRT
<213>  Artificial

<220>
<223>  Synthetic self-assembling peptide

<400>  17

Tyr Gln Tyr Gln Tyr Gln Tyr Gln Tyr Gln Tyr Gln Tyr Gln Tyr Gln
1               5                   10                  15


<210>  18
<211>  16
<212>  PRT
<213>  Artificial

<220>
<223>  Synthetic self-assembling peptide

<400>  18

His Gln His Gln His Gln His Gln His Gln His Gln His Gln His Gln
1               5                   10                  15


<210>  19
<211>  16
<212>  PRT
<213>  Artificial

<220>
<223>  Synthetic self-assembling peptide

<400>  19

Ala Asn Ala Asn Ala Asn Ala Asn Ala Asn Ala Asn Ala Asn Ala Asn
1               5                   10                  15


<210>  20
<211>  16
<212>  PRT
<213>  Artificial

<220>
<223>  Synthetic self-assembling peptide

<400>  20

Val Asn Val Asn Val Asn Val Asn Val Asn Val Asn Val Asn Val Asn
1               5                   10                  15


<210>  21
<211>  16
<212>  PRT
<213>  Artificial

<220>
<223>  Synthetic self-assembling peptide

<400>  21

Tyr Asn Tyr Asn Tyr Asn Tyr Asn Tyr Asn Tyr Asn Tyr Asn Tyr Asn
1               5                   10                  15


<210>  22
<211>  16
<212>  PRT
<213>  Artificial

<220>
<223>  Synthetic self-assembling peptide

<400>  22

His Asn His Asn His Asn His Asn His Asn His Asn His Asn His Asn
1               5                   10                  15


<210>  23
<211>  16
<212>  PRT
<213>  Artificial

<220>
<223>  Synthetic self-assembling peptide

<400>  23

Ala Asn Ala Gln Ala Asn Ala Gln Ala Asn Ala Gln Ala Asn Ala Gln
1               5                   10                  15


<210>  24
<211>  16

<212>  PRT
<213>  Artificial

<220>
<223>  synthetic self-assembling peptide

<400>  24

Ala Gln Ala Asn Ala Gln Ala Asn Ala Gln Ala Asn Ala Gln Ala Asn
1               5                   10                  15


<210>  25
<211>  16
<212>  PRT
<213>  Artificial

<220>
<223>  synthetic self-assembling peptide

<400>  25

Val Asn Val Gln Val Asn Val Gln Val Asn Val Gln Val Asn Val Gln
1               5                   10                  15


<210>  26
<211>  16
<212>  PRT
<213>  Artificial

<220>
<223>  synthetic self-assembling peptide

<400>  26

Val Gln Val Asn Val Gln Val Asn Val Gln Val Asn Val Gln Val Asn
1               5                   10                  15


<210>  27
<211>  16
<212>  PRT
<213>  Artificial

<220>
<223>  synthetic self-assembling peptide

<400>  27

Tyr Asn Tyr Gln Tyr Asn Tyr Gln Tyr Asn Tyr Gln Tyr Asn Tyr Gln
1               5                   10                  15


<210>  28
<211>  16
<212>  PRT
<213>  Artificial

<220>
<223>  synthetic self-assembling peptide

<400>  28

Tyr Gln Tyr Asn Tyr Gln Tyr Asn Tyr Gln Tyr Asn Tyr Gln Tyr Asn
1               5                   10                  15

<210>  29
<211>  16
<212>  PRT
<213>  Artificial

<220>
<223>  Synthetic self-assembling peptide

<400>  29

His Asn His Gln His Asn His Gln His Asn His Gln His Asn His Gln
1               5                   10                  15


<210>  30
<211>  16
<212>  PRT
<213>  Artificial

<220>
<223>  Synthetic self-assembling peptide

<400>  30

His Gln His Asn His Gln His Asn His Gln His Asn His Gln His Asn
1               5                   10                  15


<210>  31
<211>  18
<212>  PRT
<213>  Artificial

<220>
<223>  Synthetic self-assembling peptide

<400>  31

Ala Lys Ala Gln Ala Asp Ala Lys Ala Gln Ala Asp Ala Lys Ala Gln
1               5                   10                  15


Ala Asp



<210>  32
<211>  18
<212>  PRT
<213>  Artificial

<220>
<223>  Synthetic self-assembling peptide

<400>  32

Val Lys Val Gln Val Asp Val Lys Val Gln Val Asp Val Lys Val Gln
1               5                   10                  15


Val Asp


<210>  33
<211>  18
<212>  PRT
<213>  Artificial

<220>
<223>  Synthetic self-assembling peptide

<400>  33

Tyr Lys Tyr Gln Tyr Asp Tyr Lys Tyr Gln Tyr Asp Tyr Lys Tyr Gln
1               5                   10                  15

Tyr Asp

<210>  34
<211>  18
<212>  PRT
<213>  Artificial

<220>
<223>  Synthetic self-assembling peptide

<400>  34

His Lys His Gln His Asp His Lys His Gln His Asp His Lys His Gln
1               5                   10                  15

His Asp

<210>  35
<211>  16
<212>  PRT
<213>  Artificial

<220>
<223>  Synthetic self-assembling peptide

<400>  35

Arg Ala Arg Ala Asp Ala Asp Ala Arg Ala Arg Ala Asp Ala Asp Ala
1               5                   10                  15

<210>  36
<211>  16
<212>  PRT
<213>  Artificial

<220>
<223>  Synthetic self-assembling peptide

<400>  36

Arg Ala Asp Ala Arg Gly Asp Ala Arg Ala Asp Ala Arg Gly Asp Ala
1               5                   10                  15

<210>  37
<211>  16
<212>  PRT
<213>  Artificial

<220>
<223>  Synthetic self-assembling peptide

<400>  37

Arg Ala Glu Ala Arg Ala Glu Ala Arg Ala Glu Ala Arg Ala Glu Ala
1               5                   10                  15


<210>  38
<211>  16
<212>  PRT
<213>  Artificial

<220>
<223>  Synthetic self-assembling peptide

<400>  38

Lys Ala Asp Ala Lys Ala Asp Ala Lys Ala Asp Ala Lys Ala Asp Ala
1               5                   10                  15


<210>  39
<211>  17
<212>  PRT
<213>  Artificial

<220>
<223>  Synthetic self-assembling peptide

<400>  39

Ala Glu Ala Glu Ala His Ala His Ala Glu Ala Glu Ala His Ala His
1               5                   10                  15


Ala



<210>  40
<211>  16
<212>  PRT
<213>  Artificial

<220>
<223>  Synthetic self-assembling peptide

<400>  40

Phe Glu Phe Glu Phe Lys Phe Lys Phe Glu Phe Glu Phe Lys Phe Lys
1               5                   10                  15


<210>  41
<211>  16
<212>  PRT
<213>  Artificial

<220>
<223>  Synthetic self-assembling peptide

<400>  41

Leu Glu Leu Glu Leu Lys Leu Lys Leu Glu Leu Glu Leu Lys Leu Lys
1               5                   10                  15


<210>  42
<211>  16

<212>  PRT
<213>  Artificial

<220>
<223>  Synthetic self-assembling peptide

<400>  42

Ala Glu Ala Glu Ala Lys Ala Lys Ala Glu Ala Glu Ala Lys Ala Lys
1               5                   10                  15


<210>  43
<211>  12
<212>  PRT
<213>  Artificial

<220>
<223>  Synthetic self-assembling peptide

<400>  43

Ala Glu Ala Glu Ala Glu Ala Glu Ala Lys Ala Lys
1               5                   10


<210>  44
<211>  16
<212>  PRT
<213>  Artificial

<220>
<223>  Synthetic self-assembling peptide

<400>  44

Lys Ala Lys Ala Lys Ala Lys Ala Glu Ala Glu Ala Glu Ala Glu Ala
1               5                   10                  15


<210>  45
<211>  16
<212>  PRT
<213>  Artificial

<220>
<223>  Synthetic self-assembling peptide

<400>  45

Ala Glu Ala Glu Ala Glu Ala Glu Ala Lys Ala Lys Ala Lys Ala Lys
1               5                   10                  15


<210>  46
<211>  16
<212>  PRT
<213>  Artificial

<220>
<223>  Synthetic self-assembling peptide

<400>  46

Arg Ala Arg Ala Arg Ala Arg Ala Asp Ala Asp Ala Asp Ala Asp Ala
1               5                   10                  15

<210>  47
<211>  16
<212>  PRT
<213>  Artificial

<220>
<223>  synthetic self-assembling peptide

<400>  47

Ala Asp Ala Asp Ala Asp Ala Asp Ala Arg Ala Arg Ala Arg Ala Arg
1               5                   10                  15


<210>  48
<211>  16
<212>  PRT
<213>  Artificial

<220>
<223>  synthetic self-assembling peptide

<400>  48

Asp Ala Asp Ala Asp Ala Asp Ala Arg Ala Arg Ala Arg Ala Arg Ala
1               5                   10                  15


<210>  49
<211>  16
<212>  PRT
<213>  Artificial

<220>
<223>  synthetic self-assembling peptide

<400>  49

His Glu His Glu His Lys His Lys His Glu His Glu His Lys His Lys
1               5                   10                  15


<210>  50
<211>  20
<212>  PRT
<213>  Artificial

<220>
<223>  synthetic self-assembling peptide

<400>  50

Val Glu Val Glu Val Glu Val Glu Val Glu Val Glu Val Glu Val Glu
1               5                   10                  15


Val Glu Val Glu
            20


<210>  51
<211>  20
<212>  PRT
<213>  Artificial

<220>
<223>  synthetic self-assembling peptide

<400>  51

Arg Phe Arg Phe Arg Phe Arg Phe Arg Phe Arg Phe Arg Phe Arg Phe
1               5                   10                  15

Arg Phe Arg Phe
            20

## Description

The present invention relates to a pharmaceutical composition for repairing or replacing a damaged tissue, and a pharmaceutical composition for improving myocardial infarction, and more particularly to a pharmaceutical composition for repairing or replacing a damaged tissue, and a pharmaceutical composition for improving myocardial infarction containing peptide nanofibers and autologous stem cells.

Certain peptides are capable of self assembly when incubated in the presence of a low concentration of monovalent metal cation (U.S. Pat. No. 5,670,483; 6,548,630). Self-assembly of the peptides results in the formation of a gel-like structure that is non-toxic, non-immunogenic and relatively stable to proteases. After peptides form hydrogels, the hydrogels are stable in serum, aqueous solutions and cell culture medium. The hydrogels are capable of supporting the growth of cells, and are slowly digested when implanted in an animal's body. Thus, the hydrogels are suitable to be used as carriers for the delivery of therapeutic agents, such as platelet-derived growth factor (PDGF).

US Pat. No. 7,429,567, entitled "Sustained delivery of PDGF using self-assembling peptide nanofibers" discloses a therapeutic composition in which human PDGF is bound directly to peptides that self assemble into a biologically compatible hydrogel. When the composition of the hydrogel and the PDGF is implanted in a patient's body, the composition provides a slow, sustained release of PDGF. The composition is suitably used to treat patients who have undergone a myocardial infarction (MI), wherein MI occurs when the blood supplied to a part of the heart is interrupted and may lead to cardiomyocyte necrosis and apoptosis, while the myocardium will undergo deleterious remodeling, ultimately resulting in ventricular dilatation and pump dysfunction. When the composition is applied to treat an affected part selected from myocardial infarction, wound, damaged ligament, tendon or cartilage, or damaged nerve tissue, the method of using the composition comprises a step of administering the composition to the affected part, wherein the composition comprises a biologically compatible peptide hydrogel formed by self-assembling peptides and PDGF bound to a portion of the self-assembling peptides of the biologically compatible peptide hydrogel.

In a case that a rat model of myocardial injury is used, the nanofibers with bound PDGF can be locally injected into the border zone of an affected part of injured myocardium of a heart and are retained at the affected part for at least 14 days after coronary artery ligation. As a result, cardiomyocyte death is decreased and myocardial systolic function is maintained. Thus, the nanofibers with bound PDGF provide an effective method for preventing heart failure after myocardial infarction. However, PDGF only can promote the survival of few live cardiomyocytes remaining in the border zone of the affected part, but cannot promote the growth of non-viable (death) cardiomyocytes in the central zone of the affected part. As a result, the total nanofibers with bound PDGF were only injected into the border zone (i.e. peripheral edge) of the affected part through three directions (equal amount for each injection) immediately after coronary artery ligation without being injected into the central zone of the affected part of heart or other zone except for the border zone. If the myocardium in the central zone of the affected part is not treated, the cardiac performance (such as myocardial systolic and diastolic functions) of the affected part can not be efficiently improved.

As a result, it is important to think how to develop a method for improving myocardial infarction by intramyocardial or transendocardial injection of a suitable therapeutic composition, in order to solve the problems existing in the conventional therapeutic composition, as described above.

An objective of the present invention is to provide a pharmaceutical composition for repairing, or replacing a damaged tissue, which contains peptide nanofibers and autologous stem cells. These components are described in more detail below. When the pharmaceutical composition is administered to a damaged tissue, the peptide nanofibers with high biocompatibility can support the growth of cells and be slowly digested. Hence, the peptide nanofibers contained in the pharmaceutical composition can be used as carriers for delivering autologous stem cells. When the autologous stem cells are used with peptide nanofibers, the autologous stem cells within the damaged tissue can improve the therapeutic effect.

Another objective of the present invention is to provide a pharmaceutical composition for improving myocardial infarction, which contains peptide nanofibers with biological compatibility and autologous stem cells. These components are described in more detail below. When the pharmaceutical composition is administered to the infarcted area of the infarcted myocardium of a heart through intramyocardial or transendocardial injection, the peptide nanofibers can support the structure of the infarcted area. Hence, the peptide nanofibers contained in the pharmaceutical composition can attenuate adverse cardiac remodeling and dysfunction after acute infarction, while the intramyocardial or transendocardial injection of peptide nanofibers also can improve post-infarction diastolic functions and the cardiac performance. Thus, the compositions of the invention can be considered as compositions for improving myocardial function after infarction and being treatments for myocardial infarction, i.e. the compositions are of use in the treatment of myocardial infarction.

In addition, the peptide nanofibers in the infarcted area of the heart can be further used to fix and retain autologous peripheral blood stem cells (PBSCs) carried by blood flowing through the infarcted area of myocardium tissue, or in situ endothelial stem cells of an injured heart of a patient after the pharmaceutical composition is administered, so as to be also advantageous to prevent heart failure after myocardial infarction and increase the myocardial angiogenesis, the myocardial capillary density and potential myogenesis.

Furthermore, when the pharmaceutical composition of the present invention is administered to the infarcted area of the infarcted myocardium of a heart, the autologous stem cells retained within the infarcted area can be increased and the retention time of the autologous stem cells for cell therapy can be elongated. Thus, not only the pathological ventricular remodeling and the diastolic dysfunction can be efficiently prevented, but also the myocardial viability and the systolic functions can be substantially improved, while the therapeutic myocardial angiogenesis, the myocardial capillary density and potential myogenesis can be enhanced.

To achieve the above objectives, the present invention provides a pharmaceutical composition for repairing, or replacing a damaged tissue, which comprises: a biologically compatible peptide hydrogel comprising at least one self-assembling peptide nanofiber, wherein the self-assembling peptide nanofibers are structurally compatible to each other; and at least one cell selected from the group consisting of autologous stem cells, progenitor cells, committed cells, and mature somatic cells.

Put in another way, the pharmaceutical compositions of the invention are for use in the repair, or replacement of a damaged tissue and also for use in methods for repairing, or replacing a damaged tissue.

The present invention also provides the use of the pharmaceutical compositions of the invention in the manufacture of a medicament for repairing, or replacing a damaged tissue.

The present invention also provides the use of at least one biologically compatible self-assembling peptide nanofiber, wherein the self-assembling peptide nanofibers are structurally compatible to each other; and at least one cell selected from the group consisting of autologous stem cells, progenitor cells, committed cells, and mature somatic cells in the manufacture of a medicament for repairing, or replacing a damaged tissue. Said medicament may be any of the pharmaceutical compositions described herein.

In addition, the present invention also provides a pharmaceutical composition for improving myocardial infarction, which comprises: a biologically compatible peptide hydrogel comprising at least one self-assembling peptide nanofiber, wherein the self-assembling peptide nanofibers are structurally compatible to each other; and at least one cell selected from the group consisting of autologous stem cells, progenitor cells, committed cells, and mature somatic cells.

Put in another way, the pharmaceutical compositions of the invention are for use in improving myocardial infarction and also for use in methods for improving myocardial infarction.

The present invention also provides the use of the pharmaceutical compositions of the invention in the manufacture of a medicament for improving myocardial infarction.

The present invention also provides the use of at least one biologically compatible self-assembling peptide nanofiber, wherein the self-assembling peptide nanofibers are structurally compatible to each other; and at least one cell selected from the group consisting of autologous stem cells, progenitor cells, committed cells, and mature somatic cells in the manufacture of a medicament for improving myocardial infarction. Said medicament may be any of the pharmaceutical compositions described herein.

According to the pharmaceutical compositions of the present invention, the self-assembling peptide nanofibers are biologically compatible, and complementary and structurally compatible to each other, preferably. In addition, the length of the self-assembling peptide nanofibers is preferably 8-200 amino acids.

In addition, according to the pharmaceutical composition for improving myocardial infarction, the pharmaceutical composition can be administered to an entire infarcted area of myocardium tissue with myocardial infarction by intramyocardial or transendocardial injection.

In one aspect of the present invention, the biologically compatible peptide nanofibers may be prepared by: dissolving powders of the self-assembling peptide nanofibers in a buffer solution; and mixing the powders of the self-assembling peptide nanofibers with the buffer solution, so as to obtain a hydrogel solution of the biologically compatible peptide nanofibers. Herein, the powders of the self-assembling peptide nanofibers can be mixed with the buffer solution by any mixing manner used in the art. Preferably, the powders of the self-assembling peptide nanofibers are mixed with the buffer solution by sonication.

In one aspect of the present invention, the percentage of the self-assembling peptide nanofibers in the solution may be 0.1-10 % by weight of the solution, preferably 0.5-5 % by weight, and more preferably 1.0 % by weight. In addition, the volume of the solution of the biologically compatible peptide hydrogel is preferably 0.1-10 ml, and more preferably 1-5 ml.

In one aspect of the present invention, the buffer solution is added with monovalent metal cation of a concentration sufficient to promote the self-assembly of the self-assembling peptide nanofibers, wherein the monovalent metal cation can be any alkali or alkaline-earth cations. Preferably, the monovalent metal cation is selected from the group consisting of lithium (Li⁺), sodium (Na⁺) and potassium (K⁺).

In one aspect of the present invention, the pharmaceutical composition may be injected to a plurality of delivery sites in the entire infarcted area of myocardium tissue, wherein the number of the delivery sites is preferably ranged between 5 and 100, and more preferably 10-50.

In one aspect of the present invention, the infarcted area of myocardium tissue can be anywhere of the heart, but preferably the interventricular septum and ventricular free wall of both ventricles.

In one aspect of the present invention, the pharmaceutical composition may be prepared by mixing the solution of the biologically compatible peptide nanofibers with autologous stem cells having 10⁵-10'° cells, preferably 10⁶-10⁹ cells, and more preferably 10⁸-10⁹ cells.

In one aspect of the present invention, the damaged tissue can be included in organs and organ systems. The examples of the organs and the organ systems can be: (1) circulatory system (such as heart and blood vessels); (2) digestive system (such as salivary glands, esophagus, stomach, liver, gallbladder, pancreas, intestines, rectum and anus); (3) endocrine system (such as the hypothalamus, pituitary or pituitary gland, pineal body or pineal gland, thyroid, parathyroid and adrenals); (4) excretory system (such as kidneys, ureters, bladder and urethra); (5) integumentary system (skin); (6) lymphatic system (tonsils, thymus and spleen); (7) muscular system (skeletal muscles and smooth muscles); (8) nervous system (such as brain, spinal cord, peripheral nerves); (9) reproductive system (such as ovaries, fallopian tubes, uterus, vagina, mammary glands, testes, vas deferens, seminal vesicles, prostate and penis); (10) respiratory system (such as pharynx, larynx, trachea, bronchi, lungs and diaphragm); (11) skeletal system (such as bones, cartilage, ligaments and tendons); (12) eye, eyelid, nose, face (chin), lip, jaw, breast, ear, and any other tissues for intervention.

In addition, the damaged tissue can be necrosis, apoptosis, autophagy or any other damages caused by the following reasons: (1) ischemia, such as an infarction (i.e. blockage of blood flow to muscular or cardiac tissue) which causes tissue damage due to lack of oxygen; (2) inflammation; (3) infection; (4) trauma, such as skin burning or scraping; and (5) any other reason leading to tissue loss, defect or dysfunction.

In one aspect of the present invention, the autologous stem cells may be selected from autologous adult stem cells or autologous induced pluripotent or multipotent stem cells. The examples of the autologous stem cells can be: (1) autologous adult stem cells such as bone marrow mononuclear cells, umbilical cord blood or placental stem cells, peripheral blood stem cells (PBSCs), dental stem cells, amniotic fluid stem cells, adipose stem cells and any other somatic stem cells isolated from tissues; (2) autologous induced pluripotent stem cells (iPS), which are transformed into stem cells with the potential of differentiation using viral or non-viral transfection methods or pharmacological inducers; (3) autologous somatic progenitor cells, for example, the endothelial progenitor cells separated from fats, heart, lungs, vessels, bone marrow or any other tissues; and (4) autologous committed or mature somatic cells which are beneficial to wound healing, angiogenesis, tissue repair (such as endothelial cell and fibroblast).

In one aspect of the present invention, the autologous induced pluripotent or multipotent stem cells may be selected from autologous somatic cells, which are transformed into stem cells with the potential of differentiating into specific committed cells. The specific example of the committed cells can be: (1) cardiomyocytes, vascular smooth muscle cells, endothelial cells, pacemaker cells, and cardiac fibroblasts, as well as other cells for cardiac regeneration; (2) esophagus cells, smooth muscle cells, endothelial cells, peptic cells, parietal cells, hepatocytes, gallbladder cells, pancreas cells, and intestine cells, as well as other cells for digestive system regeneration; (3) hypothalamus cells, pituitary cells, pituitary cells, thyroid cells, and parathyroid cells, and adrenals, as well as other cells for endocrine system regeneration; (4) renal cells, smooth muscle cells, and bladder cell, as well as other cells for excretory regeneration; (5) epithelial cell, and fibroblast, as well as other cells for integumentary regeneration; (6) tonsil cells, thymus cells, and spleen cells, as well as other cells for lymphatic regeneration; (7) skeletal muscle cells, smooth muscle cells, and endothelial cells, as well as other cells for muscular regeneration; (8) brain cells, myeloid cells, and nervous cells, as well as other cells for nervous regeneration; (9) ovary cells, uterus cells, vagina cells, testes cells, and prostate cells, as well as other cells for reproductive regeneration; (10) pulmonary cells, smooth muscle cells, endothelial cells, and ciliated cells, as well as other cells for respiratory system regeneration; (11) osteoblast, osteoclast, cartilage cell, ligament cells, and tendon cells, as well as other cells for skeletal regeneration.

In one aspect of the present invention, the biologically compatible peptide nanofibers in the infarcted area may be further used to fasten and retain autologous peripheral blood stem cells (PBSCs) carried by blood flowing through the infarcted area of myocardium tissue or in situ endothelial or stem cells after the pharmaceutical composition (comprising the autologous stem cells) is administered.

In one aspect of the present invention, the self-assembling peptide nanofibers are preferably 12-32 amino acids in length, more preferably 12-20 amino acids in length, and most preferably 16 amino acids in length. In addition, the self-assembling peptide nanofibers have alternating hydrophobic and hydrophilic amino acids.

In one aspect of the present invention, the self-assembling peptide nanofibers may be homogeneous.

In one aspect of the present invention, the self-assembling peptide nanofibers may be selected from the group consisting of:
AKAKAEAEAKAKAEAE, (SEQ ID NO 1)
AKAEAKAEAKAEAKAE, (SEQ ID NO 2)
EAKAEAKAEAKAEAKA, (SEQ ID NO 3)
KAEAKAEAKAEAKAEA, (SEQ ID NO 4)
AEAKAEAKAEAKAEAK, (SEQ ID NO 5)
ADADARARADADARAR, (SEQ ID NO 6)
ARADARADARADARAD, (SEQ ID NO 7)
DARADARADARADARA, (SEQ ID NO 8)
RADARADARADARADA, (SEQ ID NO 9)
ADARADARADARADAR, (SEQ ID NO 10)
ARADAKAE ARADAKAE, (SEQ ID NO 11)
AKAEARADAKAKARAD, (SEQ ID NO 12)
ARAKADAEARAKADAE, (SEQ ID NO 13)
AKARAEADAKARAEAD, (SEQ ID NO 14)
AQAQAQAQAQAQAQAQ, (SEQ ID NO 15)
VQVQVQVQVQVQVQVQ, (SEQ ID NO 16)
YQYQYQYQYQYQYQYQ, (SEQ ID NO 17)
HQHQHQHQHQHQHQHQ, (SEQ 10 NO 18)
ANANANANANANANAN, (SEQ ID NO 19)
VNVNVNVNVNVNVNVN, (SEQ ID NO 20)
YNYNYNYNYNYNYNYN, (SEQ ID NO 21)
HNHNHNHNHNHNHNHN, (SEQ ID NO 22)
ANAQANAQANAQANAQ, (SEQ ID NO 23)
AQANAQANAQANAQAN, (SEQ ID NO 24)
VNVQVNVQVNVQVNVQ, (SEQ ID NO 25)
VQVNVQVNVQVNVQVN, (SEQ ID NO 26)
YNYQYNYQYNYQYNYQ, (SEQ ID NO 27)
YQYNYQYNYQYNYQYN, (SEQ ID NO 28)
HNHQHNHQHNHQHNHQ, (SEQ ID NO 29)
HQHNHQHNHQHNHQHN, (SEQ ID NO 30)
AKAQADAKAQADAKAQAD, (SEQ ID NO 31)
VKVQVDVKVQVDVKVQVD, (SEQ ID NO 32)
YKYQYDYKYQYDYKYQYD, (SEQ ID NO 33)
HKHQHDHKHQHDHKHQHD, (SEQ ID NO 34)
RARADADARARADADA, (SEQ ID NO 35)
RADARGDARADARGDA, (SEQ ID NO 36)
RAEARAEARAEARAEA, (SEQ ID NO 37)
KADAKADAKADAKADA, (SEQ ID NO 38)
AEAEAHAHAEAEAHAHA, (SEQ ID NO 39)
FEFEFKFKFEFEFKFK, (SEQ ID NO 40)
LELELKLKLELELKLK, (SEQ ID NO 41)
AEAEAKAKAEAEAKAK, (SEQ ID NO 42)
AEAEAEAEAKAK, (SEQ ID NO 43)
KAKAKAKAEAEAEAEA, (SEQ ID NO 44)
AEAEAEAEAKAKAKAK, (SEQ ID NO 45)
RARARARADADADADA, (SEQ ID NO 46)
ADADADADARARARAR, (SEQ ID NO 47)
DADADADARARARARA, (SEQ ID NO 48)
HEHEHKHKHEHEHKHK, (SEQ ID NO 49)
VEVEVEVEVEVEVEVEVEVE, (SEQ ID NO 50)
RFRFRFRFRFRFRFRFRFRF, (SEQ ID NO 51)

In one aspect of the present invention, the self-assembling peptide nanofibers are preferably RARADADARARADADA (SEQ ID NO 35), ADADARARADADARAR (SEQ ID NO 6), ARADARADARADARAD (SEQ ID NO 7), DARADARADARADARA (SEQ ID NO 8), RADARADARADARADA (SEQ ID NO 9), ADARADARADARADAR (SEQ ID NO 10), RARARARADADADADA (SEQ ID NO 46), ADADADADARARARAR (SEQ ID NO 47) or DADADADARARARARA (SEQ ID NO 48).

In one aspect of the present invention, the pharmaceutical compositions can be administered by (1) direct injection, for example, intra-cardiac injection by open-chest surgery or trans-endocardial injection by catheterization in the case of application for cardiac repair, or injection into the aforementioned tissues; and (2) superficial coating or patching, for example, smearing over the wound (such as skin) or patching the infarcted tissue with peptide nanofibers consisted as a sheet or a laminate (such as endocardium). Hence, the pharmaceutical compositions of the present invention can be formulated into an injection, a superficial coating, or a patch.

In preferred embodiments the present invention is related to a method for improving myocardial infarction by intramyocardial or transendocardial injection of peptide nanofibers (or peptide nanofibers with autologous stem cells), wherein a pharmaceutical composition comprising biologically compatible peptide hydrogels formed by self-assembling peptide nanofibers can be injected into an entire infarcted area of infarcted myocardium of a heart for supporting the myocardial structure of the entire infarcted area and improving the cardiac performance. Preferably, the pharmaceutical composition can further comprise at least one type of autologous stem cells which are mixed with the self-assembling peptide nanofibers and attached thereto for increasing the therapeutic myocardial angiogenesis, the myocardial capillary density and potential myogenesis. Thus, the cardiac performance of the entire infarcted area of infarcted myocardium can be improved and enhanced.

The biologically compatible peptide hydrogels formed by the self-assembling peptide nanofibers can be singly used for supporting the myocardial structure. If necessary, the biologically compatible peptide nanofibers can be mixed with an active component. For example, in the present invention, the autologous stem cells, and particularly the autologous bone marrow cells, can be mixed with and tightly attached to a portion of some self-assembling peptide nanofibers. This allows the biologically compatible peptide nanofibers to be used as a drug delivery of immobilized autologous stem cells, so that the biologically compatible peptide nanofibers can be implanted in vivo without rapidly losing the autologous stem cells. Using a sexually matured Lanyu mini-pig model of myocardial injury, the biologically compatible peptide nanofibers bound with the autologous stem cells can be locally injected into the entire infarcted area of injured myocardium and are retained at a plurality of delivery sites in the infarcted area for at least 14 days after experimental coronary artery ligation. As a result, cardiomyocyte death can be apparently decreased and myocardial function can be maintained. Thus, the biologically compatible peptide nanofibers bound with the autologous stem cells provide a more effective method for preventing heart failure after myocardial infarction. Furthermore, the biologically compatible peptide nanofibers in the infarcted area can be selectively used to further fasten and retain autologous peripheral blood stem cells (PBSCs) carried by blood flow through the infarcted area of myocardium tissue or in situ endothelial or stem cells of an injured heart of a patient or an animal model for therapeutic angiogenesis in the heart after the pharmaceutical composition is administered, so as to be also advantageous to prevent heart failure after myocardial infarction and increase myocardial angiogenesis, myocardial capillary density and potentially myogenesis.

In one preferable embodiment of the present invention, a biologically compatible peptide hydrogel constructed by self-assembling peptide nanofibers is applied to intramyocardial or transendocardial injection, wherein the term "biologically compatible" indicates that the hydrogel is non-toxic and can be safely implanted in a patient, while the term "hydrogel" in the present invention refers to a three dimensional gel-like structure. The self-assembling peptide nanofibers should be 8-200 amino acids in length and have alternating hydrophobic and hydrophilic amino acids. In addition, the peptide nanofibers are complementary for forming ionic or hydrogen bonds with one another, and are also structurally compatible, wherein the peptide chains bound to one another can maintain a distance less than about 3 angstroms throughout their length from one another. In general, 0.1-10%, more preferably 0.5-5 %, and still more preferably about 1 % by weight of the peptide nanofibers that assemble into the hydrogel should be bound directly to at least one type of autologous stem cells. The term "bound directly" as used herein means that there is no linker molecule or other molecules dispersed between the autologous stem cells and the peptide nanofibers. The term also requires that there be some type of physical interaction between the autologous stem cells and the peptide nanofibers (e.g. an ionic bond, hydrophobic interaction and etc.), so as to prevent the autologous stem cells from separating from the peptide nanofibers of the hydrogel in aqueous medium. Ionic bonds would form between acidic and basic amino acid side chains. The hydrophilic basic amino acids include Lys (K), Arg (R) and His (H). The hydrophilic acidic amino acids are Glu (E) and Asp (D). Ionic bonds would form between an acidic residue on one peptide and a basic residue on another. Amino acids that form hydrogen bonds are Asn (N) and Gln (Q). Hydrophobic amino acids that may be incorporated into peptide nanofibers include Ala (A), Val (V), lie (I), Met (M), Phe (F), Tyr (Y), Trp (W), Ser (S), Thr (T), and Gly (G).

The present invention is also related to administering a pharmaceutical composition to an infarcted area of myocardium tissue of an injured heart of a patient or an animal model with myocardial infarction. It should be noted that the term "infarcted area" in the present invention refers to an affected part of myocardium tissue where myocardial infarction occurs, wherein most of cardiomyocytes in the central zone and the border zone of the affected part are almost seriously injured or died, while a few of cardiomyocytes in the border zone of the affected part are slightly injured and still viable. The term "entire infarcted area" in the present invention refers to the infarcted area considerably comprises a plurality of delivery sites which can be injected with peptide nanofibers or peptide nanofibers with autologous stem cells, wherein the number of the delivery sites is preferably ranged between 5 and 100, and more preferably 10-50, but can be suitably varied according to the size of the infarcted area. The infarcted area of myocardium tissue is preferably a mid-left portion of myocardium tissue of an injured heart with myocardial infarction, but also can be any infarcted area of the heart without limitation thereto.

The self-assembling peptide nanofibers have been described in U.S. Pat. Nos. 5,670,483 and 6,548,630. Both of which are hereby incorporated by reference. Essentially the same procedures described therein for making and using the peptide nanofibers apply to the present invention. However, it has been found that the autologous stem cells can be non-covalently bound to the hydrogel formed by the self-assembling peptide nanofibers by simply combining the peptide nanofibers and the autologous stem cells in aqueous medium (e.g., water, saline or a buffer containing the components needed for the self assembly of peptide nanofibers). It appears that, 0.5-5% of the peptide nanofibers within hydrogels can be bound to the autologous stem cells but an upper limit is not limited thereto. In the preferred embodiment, the self-assembling peptide nanofibers used in hydrogels are between 8 and 200 amino acids in length, preferably between 12 and 32 amino acids in length, and more preferably 16 amino acids in length. Peptide nanofibers longer than about 200 amino acids in length tend to lower the solubility thereof and thus may be avoided. In addition, some of the self-assembling peptide nanofibers attached with the autologous stem cells are homogeneous. The term "homogeneous" as used in the present invention indicates that all of the peptide nanofibers forming the biologically compatible hydrogel are substantially identical. The term "heterogeneous" refers to non-identical peptide nanofibers that are used to form hydrogels. Specific peptide nanofibers that may be used to construct the hydrogels described above are selected from the group consisting of:
AKAKAEAEAKAKAEAE, (SEQ ID NO 1)
AKAEAKAEAKAEAKAE, (SEQ ID NO 2)
EAKAEAKAEAKAEAKA, (SEQ ID NO 3)
KAEAKAEAKAEAKAEA, (SEQ ID NO 4)
AEAKAEAKAEAKAEAK, (SEQ ID NO 5)
ADADARARADADARAR, (SEQ ID NO 6)
ARADARADARADARAD, (SEQ ID NO 7)
DARADARADARADARA, (SEQ ID NO 8)
RADARADARADARADA, (SEQ ID NO 9)
ADARADARADARADAR, (SEQ ID NO 10)
ARADAKAEARADAKAE, (SEQ ID NO 11)
AKAEARADAKAKARAD, (SEQ ID NO 12)
ARAKADAEARAKADAE, (SEQ ID NO 13)
AKARAEADAKARAEAD, (SEQ ID NO 14)
AQAQAQAQAQAQAQAQ, (SEQ ID NO 15)
VQVQVQVQVQVQVQVQ, (SEQ ID NO 16)
YQYQYQYQYQYQYQYQ, (SEQ ID NO 17)
HQHQHQHQHQHQHQHQ, (SEQ ID NO 18)
ANANANANANANANAN, (SEQ ID NO 19)
VNVNVNVNVNVNVNVN, (SEQ ID NO 20)
YNYNYNYNYNYNYNYN, (SEQ ID NO 21)
HNHNHNHNHNHNHNHN, (SEQ ID NO 22)
ANAQANAQANAQANAQ, (SEQ ID NO 23)
AQANAQANAQANAQAN, (SEQ ID NO 24)
VNVQVNVQVNVQVNVQ, (SEQ ID NO 25)
VQVNVQVNVQVNVQVN, (SEQ ID NO 26)
YNYQYNYQYNYQYNYQ, (SEQ ID NO 27)
YQYNYQYNYQYNYQYN, (SEQ ID NO 28)
HNHQHNHQHNHQHNHQ, (SEQ ID NO 29)
HQHNHQHNHQHNHQHN, (SEQ ID NO 30)
AKAQADAKAQADAKAQAD, (SEQ ID NO 31)
VKVQVDVKVQVDVKVQVD, (SEQ ID NO 32)
YKYQYDYKYQYDYKYQYD, (SEQ ID NO 33)
HKHQHDHKHQHDHKHQHD, (SEQ ID NO 34)
RARADADARARADADA, (SEQ ID NO 35)
RADARGDARADARGDA, (SEQ ID NO 36)
RAEARAEARAEARAEA, (SEQ ID NO 37)
KADAKADAKADAKADA, (SEQ ID NO 38)
AEAEAHAHAEAEAHAHA, (SEQ ID NO 39)
FEFEFKFKFEFEFKFK, (SEQ ID NO 40)
LELELKLKLELELKLK, (SEQ ID NO 41)
AEAEAKAKAEAEAKAK, (SEQ ID NO 42)
AEAEAEAEAKAK, (SEQ ID NO 43)
KAKAKAKAEAEAEAEA, (SEQ ID NO 44)
AEAEAEAEAKAKAKAK, (SEQ ID NO 45)
RARARARADADADADA, (SEQ ID NO 46)
ADADADADARARARAR, (SEQ ID NO 47)
DADADADARARARARA, (SEQ ID NO 48)
HEHEHKHKHEHEHKHK, (SEQ ID NO 49)
VEVEVEVEVEVEVEVEVEVE, (SEQ ID NO 50) and
RFRFRFRFRFRFRFRFRFRF (SEQ ID NO 51).

It should be noted that each of the peptide nanofibers listed above includes a repeating sequence and that additional repeats can be included to extend the length of the peptide nanofibers without affecting the property of self-assembly. For example, the peptide AKAKAEAEAKAKAEAE (SEQ ID NO: 1) has the repeating sequence AKAKAEAE and can be expressed as (AKAKAEAE)ₙ, wherein n=2. Longer peptide nanofibers capable of self assembly can be made by increasing n, but the total number of amino acids in the final peptide cannot exceed 200.

Other peptide nanofibers expressed in this manner and useful in the invention are: (AKAKAEAE)ₙ, wherein n=1-25; (AKAE)ₙ, wherein n=2-50; (EAKA)ₙ, wherein n=2-50; (KAEA)ₙ, wherein n=2-50; (AEAK)ₙ, wherein n=2-50; (ADADARAR)ₙ, wherein n=1-25; (ARAD)ₙ, wherein n=2-50; (DARA)ₙ, wherein n=2-50; (RADA)ₙ, wherein n=2-50; (ADAR)ₙ, wherein n=2-50; (ARADAKAE)ₙ, wherein n=1-25; (AKAEARAO}ₙ, wherein n=1-25; (ARAKADAE)ₙ, wherein n=1-25; (AKARAEAD)ₙ, wherein n=1-25; (AQ)ₙ, wherein n=4-100; (VQ)ₙ, wherein n=4-100; (YQ)ₙ, wherein n=4-100; (HQ)ₙ, wherein n=4-100; (AN)ₙ, wherein n=4-100; (VN)ₙ, wherein n=4-100; (YN)ₙ, wherein n=4-100; (HN)ₙ, wherein n=4-100; (ANAQ)ₙ, wherein n=2-50; (AQAN)ₙ, wherein n=2-50; (VNVQ)ₙ, wherein n=2-50; (VQVN)ₙ, wherein n=2-50; (YNYQ)ₙ, wherein n=2-50; (YQYN)ₙ, wherein n=2-50; (HNHQ)ₙ, wherein n=2-50; (HQHN)ₙ, wherein n=2-50; (AKAQAD)ₙ, wherein n=2-33; (VKVQVD)ₙ, wherein n=2-33; (YKYQYD)ₙ, wherein n=2-33; (HKHQHD)ₙ, wherein n=2-33; (RARADADA)ₙ, wherein n=1-25; (RADARGDA)ₙ, wherein n=1-25; (RAEA)ₙ, wherein n=2-50; (KADA)ₙ, wherein n=2-50; (AEAEAHAH)ₙ, wherein n=1-25; (FEFEFKFK)ₙ, wherein n=1-25; (LELELKLK)ₙ, wherein n=1-25; (AEAEAKAK)ₙ, wherein n=1-25; (AEAEAEAEAKAK)ₙ, wherein n=1-16; (KAKAKAKAEAEAEAEA)ₙ, wherein n=1-12; (AEAEAEAEAKAKAKAK)ₙ, wherein n=1-12; (RARARARADADADADA)ₙ, wherein n=1-12; (ADADADADARARARAR), wherein n=1-12; (DADADADARARARARA)ₙ, wherein n=1-12; (HEHEHKHK)ₙ, wherein n=1-25; (VE)ₙ, wherein n=4-100; and (RF)ₙ, wherein n=4-100.

Preferred peptide nanofibers are those having the following repeating structures:
(RARADADA)ₙ, wherein n=1-10, preferably n=2-4, and more preferably n=2, i.e.
RARADADARARADADA, (SEQ ID NO 35); (ADADARAR)ₙ, wherein n=1-25, e.g.
ADADARARADADARAR, (SEQ ID NO 6); (ARAO)ₙ, wherein n=2-50, e.g.
ARADARADARADARAD, (SEQ ID NO 7); (DARA)ₙ, wherein n=2-50, e.g.
DARADARADARADARA, (SEQ ID NO 8); (RADA)ₙ, wherein n=2-50, e.g.
RADARADARADARADA, (SEQ ID NO 9); (ADAR)ₙ, wherein n=2-50, e.g.
ADARADARADARADAR, (SEQ ID NO 10); (RARARARADADADADA)ₙ, wherein
n=1-12, e.g. RARARARADADADADA, (SEQ ID NO 46); (ADADADADARARARAR),
wherein n=1-12, e.g. ADADADADARARARAR, (SEQ ID NO 47);
(DADADADARARARARA)ₙ, wherein n=1-12, e.g. DADADADARARARARA, (SEQ ID NO 48).

In the present invention, the self-assembling peptide nanofibers must also be structurally compatible for maintaining an essentially constant distance between one another when binding one another to self-assemble the hydrogel. Inter-peptide distance can be calculated for each ionized or hydrogen bonding pair by taking the sum of the number of unbranched atoms on the side-chains of each amino acid in the pair. For example, lysine has five unbranched atoms on its side chains, and glutamic acid has four unbranched atoms on its side chains. An interaction between these two residues on different peptide nanofibers would result in an interpeptide distance of nine atoms. In a peptide containing only repeating units of EAK, all of the ion pairs would involve lysine (K) and glutamate (E), so that a constant interpeptide distance would be maintained. Thus, these peptide nanofibers would be structurally complementary to one another. Peptide nanofibers in which the variation in interpeptide distance is more than one atom (about 3-4 angstroms) will not properly form a hydrogel structure. For example, if two bound peptide nanofibers have ion pairs with a nine-atom spacing and other ion pairs with a seven-atom spacing, the requirement of structural complementarity can not have been met, wherein other discussion of complementarity and structural compatibility can be found in U.S. Pat. No. 5,670,483 and 6,548,630. The definitions used therein and examples provided can be applied equally to the present invention.

It should also be noted that the hydrogels may be formed from either a homogeneous mixture of peptide nanofibers or a heterogeneous mixture of peptide nanofibers. The term "homogeneous" in the present invention means peptide nanofibers that are identical to one another. The term "heterogeneous" indicates peptide nanofibers that bind to one another but which are structurally different from one another. Regardless of whether homogenous or heterogeneous peptide nanofibers are used, the requirements with respect to the arrangement of amino acids, length, complementarity, and structural compatibility must apply. In addition, it should be noted that the carboxyl and amino groups of the terminal residues of peptide nanofibers can either be protected or not protected using standard groups.

The self-assembling peptide nanofibers of the present invention can be formed by solid-phase peptide synthesis using standard N-tert-butyoxycarbonyl (t-Boc) chemistry and cycles using n-methylpyrolidone chemistry. Once peptide nanofibers have been synthesized, they can be purified using procedures such as high pressure liquid chromatography on reverse-phase columns. Purity may also be assessed by HPLC (high-performance liquid chromatography) and the presence of a correct composition can be determined by amino acid analysis.

The self-assembling peptide nanofibers described herein will not form hydrogels in water, but will self-assemble in an aqueous solution (such as a buffer solution) containing monovalent metal cation of a low concentration. The order of effectiveness of these cations is Li⁺ > Na⁺ > K⁺ > Cs⁺ (U.S. Pat. No. 6,548,630). A concentration of monovalent metal cation of 5 mM is sufficient for peptide nanofibers to self-assemble, and the concentration as high as 5 M still can be effective. The anion associated with the monovalent metal cation is not critical to the present invention and can be hydroxide, acetate, chloride, sulfate, phosphate, etc. If the autologous stem cells are used, the autologous stem cells will bind to the peptide nanofibers at low salt concentration and will remain bound at concentrations sufficient to induce self assembly.

The initial concentration of peptide nanofibers will influence the final size and thickness of the hydrogel formed therefrom. In general, when the peptide concentration is increased, the extent of hydrogel formation can be expanded. The hydrogel can be formed in peptide concentration as low as 0.1-10 % by weight of the solution, preferably 0.5-5 % by weight, and more preferably 1.0 % by weight. However, the hydrogel is preferably formed at a higher initial peptide concentration, such as 1.0 % by weight (10 mg/ml). Moreover, it is generally better to form the hydrogel by adding the peptide nanofibers to a salt solution or buffer solution rather than adding salt or buffer to a peptide solution.

The formation of the hydrogel is relatively unaffected by pH or by temperature. Nevertheless, pH should be maintained below 12 and temperatures should generally be in the range of 4-90 C. Monovalent metal cation at a concentration of 5 mM is sufficient for peptide nanofibers to assemble into the hydrogel. Hydrogel formation may be observed by simple visual inspection and this can be aided, if desired, with stains such as Congo Red. The integrity of the hydrogel can also be observed microscopically, with or without stain.

Stem cells (such as mesenchymal stem cells) can be found in the bone marrow or in other autologous tissues of adult humans. Stem cells have the potential to differentiate and develop into mature cells of fat, cartilage, bone, tendon, nerve, muscle (such as cardiomyocytes or smooth muscle cells, SMCs) or endothelial cells (EC). In the present invention, stem cells can be isolated from human autologous bone marrow, fat, umbilical cord blood, placenta and etc., and transferred into the entire infarcted area of an infarcted myocardium of a heart via local intramyocardial or transendocardial injections, so as to grow and reproduce and even still maintain the stem cell capabilities. Autologous bone marrow mononuclear cells or autologous umbilical cord blood stem cells isolated as described above is advantageous to increase the therapeutic myocardial angiogenesis, the myocardial capillary density and potential myogenesis in the infarcted myocardium.

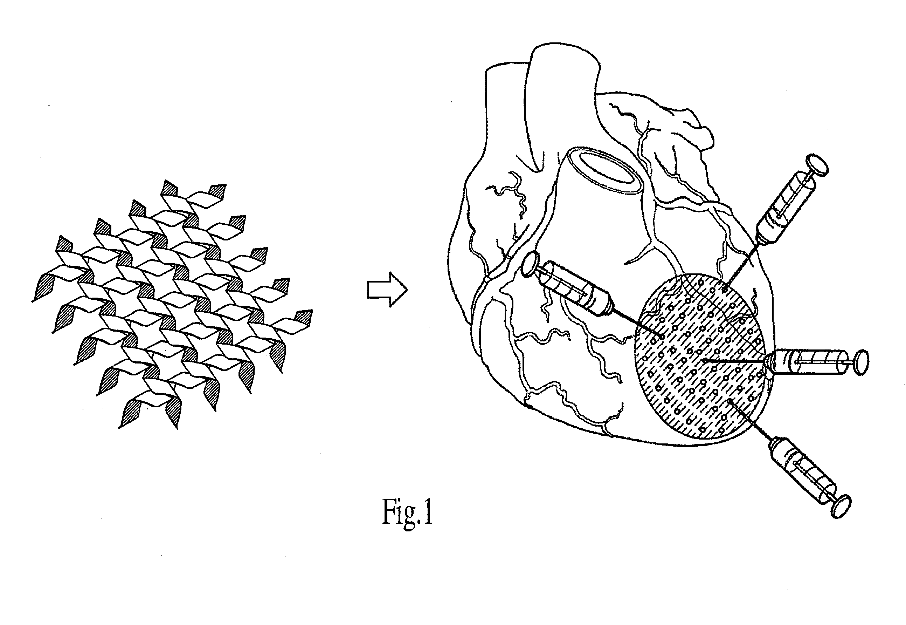

The structure and the technical means adopted by the present invention to achieve the above and other objectives can be best understood by referring to the following detailed description of the preferred non-limiting embodiments and the accompanying drawings, wherein
Fig. 1 is a schematic view of intramyocardial injection of self-assembling peptide nanofibers (NFs) into infarcted myocardium according to a preferred embodiment of the present invention, wherein sexually matured Lanyu mini-pig models of experimental myocardial infarction (MI) are treated with peptide NF injections, and the peptide NFs form a gel-like (i.e. hydrogel) structure after sonication and become consolidated after intramyocardial injections, and wherein the mid-left anterior descending coronary artery is permanently ligated, followed with a total of 2ml peptide NF injection in 40 delivery sites of the entire infarcted areas of the infarcted myocardium; and

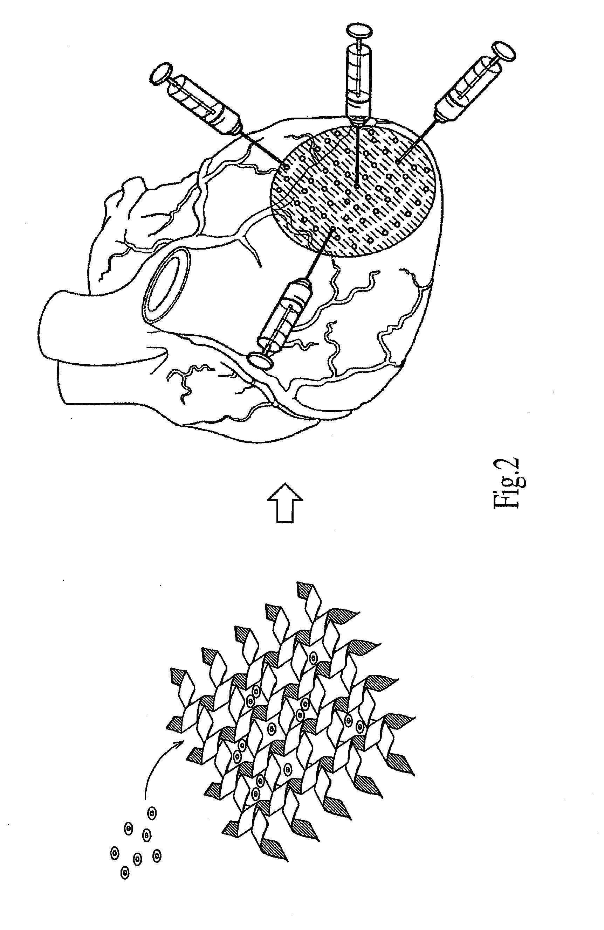

Fig. 2 is a schematic view of intramyocardial injection of self-assembling peptide nanofibers with autologous stem cells into infarcted myocardium according to another preferred embodiment of the present invention, similar to Fig. 1, wherein sexually matured Lanyu mini-pig models of experimental MI are treated with peptide NF injections with autologous bone marrow mononuclear cells, and the peptide NFs form a hydrogel structure after sonication and become consolidated after intramyocardial injections, and wherein the mid-left anterior descending coronary
artery is permanently ligated, followed with a total of 2ml peptide NF injections in 40 delivery sites of the entire infarcted areas of the infarcted myocardium.

### SEQUENCE LISTING FREE TEXT

For SEQ ID Nos 1 to 51
<223> Synthetic self-assembling peptide

### EXAMPLE 1

Referring now to Fig. 1, a method for improving myocardial infarction by intramyocardial injection of peptide nanofibers (or peptide nanofibers with autologous stem cells) according to a preferred embodiment of the present invention is illustrated, wherein the method comprises steps of: providing a pharmaceutical composition comprising a biologically compatible peptide hydrogel formed by a plurality of self-assembling peptide nanofibers with 8-200 amino acids in length, wherein the self-assembling peptide nanofibers having alternating hydrophobic and hydrophilic amino acids are complementary and structurally compatible to one another; and administering the pharmaceutical composition to an infarcted area of myocardium tissue with myocardial infarction by intramyocardial or transendocardial injection. As shown, an intramyocardial injection of self-assembling peptide nanofibers (NFs) into infarcted myocardium is carried out according to the preferred embodiment of the present invention, wherein sexually matured Lanyu mini-pigs model of experimental myocardial infarction (MI) are treated with peptide NFs injection, and the peptide NFs forms a gel-like (i.e. hydrogel) structure after sonication and becomes consolidated after intramyocardial injection, and wherein the mid-left anterior descending coronary artery is permanently ligated, followed with 50 pl peptide NFs injection in 40 delivery sites of the entire infarcted areas of the infarcted myocardium (total dose: 2 ml). The method of the preferred embodiment will be described more detailed hereinafter.

In the preferred embodiment of the present invention, the peptide nanofibers solution contains peptide nanofibers which can form a biologically compatible peptide hydrogel, wherein the biologically compatible peptide hydrogel is prepared by: dissolving powders of the self-assembling peptide nanofibers in a buffer solution, such as pH 7.4 phosphate buffered saline (PBS) solution; and mixing the powders of the self-assembling peptide nanofibers with the buffer solution by sonication (100 W, 10 mins), so as to obtain a hydrogel solution of the biologically compatible peptide hydrogel (i.e. the peptide nanofibers solution), wherein the percentage of the self-assembling peptide nanofibers in the solution can be 0.1-10 % by weight of the solution, preferably 0.5-5 % by weight, and more preferably 1.0 % by weight; and wherein the volume of the solution of the biologically compatible peptide hydrogel is preferably 0.1-10 ml, and more preferably 2 ml. In the embodiment, the percentage of the self-assembling peptide nanofibers in the solution is 1.0 % by weight, and the volume of the solution of the biologically compatible peptide hydrogel is 2 ml. Meanwhile, the buffer solution (PBS) is added with monovalent metal cation compound of a concentration sufficient to promote the self-assembly of the self-assembling peptide nanofibers, wherein the monovalent metal cation is selected from the group consisting of: lithium (Li⁺), sodium (Na⁺) and potassium (K⁺). In the embodiment, the monovalent metal cation compound is sodium hydroxide (NaOH).

Besides, the pharmaceutical composition of the treatment groups is injected to a plurality of delivery sites in the entire infarcted area of myocardium tissue, respectively, wherein the number of the delivery sites is preferably ranged between 10 and 100, and more preferably 40, without limitation thereto. The plurality of delivery sites in the entire infarcted area is advantageous to increase the distribution density to support materials (e.g. peptide nanofibers). In addition, the infarcted area of myocardium tissue is preferably a mid-left portion of myocardium tissue of an injured heart with myocardial infarction, but also can be any infarcted area of the heart. The self-assembling peptide nanofibers (AcN-RARADADARARADADA-NH₂) are 16 amino acids in length, but also can be 8-200 amino acids in length, and preferably 12-32 amino acids in length. However, only if the self-assembling peptide nanofibers can construct the biologically compatible peptide hydrogel, the self-assembling peptide nanofibers can be selected from at least one type of homogeneous or heterogeneous peptide nanofibers, such as peptide nanofibers of (SEQ ID NO 1) to (SEQ ID NO 51), preferably RARADADARARADADA (SEQ ID NO 35), ADADARARADADARAR (SEQ ID NO 6), ARADARADARADARAD (SEQ ID NO 7), DARADARADARADARA (SEQ ID NO 8), RADARADARADARADA (SEQ ID NO 9), ADARADARADARADAR (SEQ ID NO 10), RARARARADADADADA (SEQ ID NO 46), ADADADADARARARAR (SEQ ID NO 47) or DADADADARARARARA (SEQ ID NO 48), and more preferably RARADADARARADADA (SEQ ID NO 35).

### Materials and Methods

A total of 40 sexually mature minipigs (approximately 5 months old, acquired from National Taitung Animal Propagation Station and housed at NCKU Animal Center.) were divided into 5 groups: sham operation, which was performed by opening the chest without coronary artery ligation (sham), MI+normal saline (NS), MI+NF_{S}, MI+autologous bone marrow mononuclear cells (MNCs), and MI+MNCs along with NFs (MNCs/NFs; n=8 in each group). MI was induced by permanent occlusion of the midleft anterior descending coronary artery immediately followed by injection of a total of 2 mL NS or 1% NFs divided among approximately 40 injections into the entire infarcted area (50 ul for each site). Freshly isolated 1x10⁸ autologous MNCs were mixed in 2 mL NS or NFs for injection, wherein the sequence of peptide NFs is AcN-RARADADARARADADA-NH₂- Cardiac functions were assessed by echocardiography before and immediately after MI and together with hemodynamic measurements through catheterization 4 weeks later.

### Results

At 28 days after MI, 68.5±2.3% peptide nanofibers were retained in the injected regions as determined by high-performance liquid chromatography. Injection of peptide nanofibers alone significantly increased both systolic and diastolic interventricular septum thickness (systole: 0.54±0.02 cm and diastole: 0.46±0.03 cm in the MI+NS group; and systole: 0.73±0.05 cm and diastole: 0.57±0.02 cm in the MI+NFs group). Furthermore, both left ventricular end diastolic volume and left ventricular end systolic volume were improved by NF injection, suggesting that this significantly prevented post-Ml left ventricular dilatation. In addition, the post-MI diastolic function was significantly improved after peptide nanofibers injection (-dP/dt: -1109.7±91.2 mm Hg/s in the MI+NS group, and -1751.0±86.9 mm Hg/s in the MI+NFs group).

Furthermore, injection of autologous bone marrow MNCs alone significantly increased LVEF, systolic interventricular septum thickness, and +dP/dt, but neither diastolic interventricular septum thickness nor -dP/dt. However, we found that the LVEF (66.0±1.0% in the sham group, 45.2±1.8% in the Ml+NS group, 48.2±2.0% in the MI+NFs group, 52.6±2.1% in the MI+MNCs group, 58.7±1.6% in the MI+MNCs/NFs group), left ventricular end diastolic pressure, left ventricular end systolic volume, left ventricular end diastolic volume, AE, +dP/dt, -dP/dt, τ, and maximum chamber elasticity were all significantly improved as a result of MNC/NF injection (Table).

**Table. Hemodynamic Parameters at 1 Month After MI**

| | | | | | MI+MNCs/NFs |
|---|---|---|---|---|---|
| Parameter | Sham (n=8) | MI+NS (n=8) | MI+NFs (n=8) | MI+MNCs (n=8) | (n=8) |
| Heart rate, beats/min | 102.4±6.3 | 94.0±4.4 | 94.0±6.7 | 95.6±8.1 | 99.4±6.8 |
| LVESP, mm Hg | 89.0±4.1 | 69.6±2.6 | 77.5±3.4 | 77.1 ±5.7 | 79.1±4.5 |
| LVEDP, mm Hg | 8.3±0.7 | 12.7±0.9 | 8.6±1.0† | 8.6±0.7† | 8.5±0.5† |
| LVESV, mL | 35.6±3.6 | 103.0±7.0 | 73.7±3.6‡^{‡} | 58.7±2.4‡§ | 51.7±4.6‡ll |
| LVEDV, mL | 96.8±6.4 | 147.5±7.0 | 120.3±7.0† | 115.0±5.7† | 113.6±6.1† |
| SV, mL | 64.9±8.6 | 44.5±5.8 | 52.0±5.0 | 56.2±4.8 | 61.8±5.0 |
| CO, mL/min | 6636.0±959.1 | 4133.3±482.2 | 5084.6±535.2 | 5247.0±433.3 | 5879.9±654.8 |
| SW, mm Hg · mL | 4870.5±532.4 | 2527.0±414.2 | 3689.1±511.4 | 4114.5±533.6 | 4567.0±809.4 |
| AE, mm Hg/mL | 1.52±0.12 | 0.89±0.07 | 1.26±0.08* | 1.12±0.09* | 1.29±0.10* |
| +dP/dt, mm Hg/s | 1852.7±82.9 | 1214.5±91.9 | 1419.4±71.8 | 1602.1 ±135.6* | 1693.7±84.7t |
| -dP/dt, mm Hg/s | -2048.8±293.1 | -1109.7±91.2 | -1751.0±86.9* | -1401.4±127.6 | -1809.6±264.3* |
| τ(Weiss method), ms | 30.8±0.8 | 42.1 ±3.4 | 33.0±1.9* | 34.5±0.9* | 32.1±1.7* |
| PRSW, mm Hg | 65.1±8.4 | 42.2±5.6 | 55.8±6.8 | 60.5±5.9 | 65.1±13.8 |
| ESPVR, mm Hg/m L | 1.6±.2 | 0.7±0.1 | 1.2±0.2 | 1.3±0.2 | 1.5±0.2 |
| EDPVR, mm Hg/mL | 0.05±0.02 | 0.06±0.02 | 0.05±0.02 | 0.06±0.02 | 0.05±0.02 |
| Eₘₐₓ, mm Hg/mL | 3.6±0.4 | 1.4±0.2 | 2.9±0.2* | 2.5±0.5 | 3.3±0.4† |
| +dP/dt-EDV, mm Hg/s/mL | 14.3±4.5 | 6.5±1.6 | 9.4±3.0 | 9.5±2.1 | 12.0±2.9 |
| Values are mean±SEM. | | | | | |
| *P<0.05 versus MI+NS. | | | | | |
| tP<0.01 versus MI+NS. | | | | | |
| ‡P<0.001 versus MI+NS. | | | | | |
| §P<0.05 versus MI+NF_{S}. | | | | | |
| P<0.01 versus MI+NF_{S}. | | | | | |
| LVESP indicates left ventricular end systolic pressure; LVEDP, left ventricular end diastolic pressure; LVESV, left ventricular end systolic volume; LVEDV, left ventricular end diastolic volume; SV, stroke volume; CO, cardiac output; SW, stroke work; AE, arterial elastance; , time constant of left ventricular pressure decay; PRSW, preload recruitable stroke work; ESPVR, end diastolic pressure-volume relationship; EDPVR, end systolic pressure-volume relationship; Eₘₐₓ, maximum chamber elasticity; EDV, end diastolic volume. | | | | | |

As described above, in the preferred embodiment of the present invention, when intramyocardial injection of peptide nanofibers into the 40 delivery sites (or delivery sites with suitable distribution density) in the entire infarcted area of injured myocardium (MI+NFs group) of an injured heart is carried out, the injections of peptide nanofibers can support the structure of the infarcted area, while the biologically compatible peptide hydrogel in the infarcted area can be used to fasten and retain autologous peripheral blood stem cells (PBSCs) carried by blood flow through the infarcted area of myocardium tissue or in situ the endothelial or stem cells for therapeutic myocardial angiogenesis, the myocardial capillary density and potential myogenesis in the heart after the pharmaceutical composition is administered. As a result, the post-infarction diastolic functions and the cardiac performance can be improved and ventricular remodeling can be prevented in a mini-pig model of coronary ligation at the mid-left anterior descending coronary artery, while growing evidence indicated that MI+NFs group also can improve related cardiac functions and attenuate adverse cardiac remodeling and dysfunction after myocardial infarction (MI).

### EXAMPLE 2

Referring now to Fig. 2, an alternative method for improving myocardial infarction by intramyocardial injection of peptide nanofibers with autologous stem cells according to another embodiment of the present invention is illustrated and comprises steps of: providing a pharmaceutical composition comprising a biologically compatible peptide hydrogel formed by a plurality of self-assembling peptide nanofibers with 8-200 amino acids in length, wherein the self-assembling peptide nanofibers having alternating hydrophobic and hydrophilic amino acids are complementary and structurally compatible to one another, and at least one type of autologous stem cells mixed with the self-assembling peptide nanofibers; and administering the pharmaceutical composition to an infarcted area of myocardium tissue with myocardial infarction by intramyocardial injection. In the embodiment, the pharmaceutical composition further comprises at least one type of autologous stem cells mixed with the self-assembling peptide nanofibers, wherein the autologous stem cells can be selected from autologous adult stem cells or induced pluripotent/multipotent stem cells, and wherein the autologous adult stem cells are preferably selected from autologous bone marrow mononuclear cells, autologous umbilical cord blood or placental stem cells, autologous peripheral blood stem cells (PBSCs), or autologous stem cells separated from fats, heart, lungs, vessels, muscles, or other adult tissues. In addition, the autologous induced pluripotent or multipotent stem cells are selected from autologous somatic cells which are transformed into stem cells with the potential of differentiating into cardiomyocytes, vascular smooth muscle cells, endothelial cells or pacemaker cells for cardiac therapy using viral or non-viral gene transfection methods or pharmacological inducers.

In the embodiment, autologous bone marrow mononuclear cells are exemplified, and the separation method thereof comprises steps of: adding 0.5 ml of heparin into two 10 ml syringes, respectively, and then drawing out autologous bone marrow tissues from an autologous bone (such as the tibia bone) of the sexually matured Lanyu mini-pigs; placing the autologous bone marrow tissues into a 50 ml tube, and diluting by 20 ml of phosphate buffered saline (PBS) containing 5 % fetal bovine serum (FBS); evenly mixing the diluted solution, and filtering through a 70 um strainer; preparing eight 10 ml tubes, and adding 5 ml of Ficoll medium into each of the tubes, respectively; slowly and averagely adding 10 ml of the diluted solution of the autologous bone marrow tissues into the Ficoll tubes without mixing the diluted solution with the Ficoll medium; centrifuging the Ficoll tubes at 2400 rpm for 15 mins; observing the location of mononuclear cell layer after centrifugation, followed by removing the supernatant above the mononuclear cell layer and collecting the mononuclear cell layer; washing the mononuclear cells three times by PBS at 1800 rpm for 2 mins; counting the cell number of the mononuclear cells and adjusting the concentration of the mononuclear cells to a suitable value after washing; and adding 2 ml of PBS + NFs (nanofibers) into the mononuclear cells having a predetermined cell number, and drawing the solution by a syringe for intramyocardial injection.

As shown in Fig. 2, an intramyocardial injection of self-assembling peptide nanofibers with the autologous bone marrow mononuclear cells (NFs+BM) into infarcted myocardium is carried out according to the embodiment, wherein sexually matured Lanyu mini-pigs model of experimental myocardial infarction (MI) is treated with peptide NFs injection containing the autologous bone marrow mononuclear cells having a predetermined cell number, and the peptide NFs forms a gel-like (i.e. hydrogel) structure after sonication and becomes consolidated after intramyocardial injection, and wherein the mid-left anterior descending coronary artery is permanently ligated, followed with 50 µl peptide NFs injection in 40 delivery sites (or delivery sites with suitable distribution density) of the entire infarcted areas of the infarcted myocardium (total dose: 2 ml). The materials and methods of the embodiment are substantially similar to that of the foregoing preferred embodiment, so that most of the detailed description will be omitted.

In the embodiment, when intramyocardial injection of peptide nanofibers with the autologous stem cells is carried out, wherein the peptide nanofibers are mixed with autologous stem cells (such as bone marrow mononuclear cells), and the mixture are applied to intramyocardial injection into the 40 delivery sites (or delivery sites with suitable distribution density) in the entire infarcted area of the infarcted myocardium of the heart, so that the autologous stem cells retained within the infarcted area can be increased and the retention time of the autologous stem cells for cell therapy can be elongated. Thus, not only the pathological ventricular remodeling and the diastolic dysfunction can be efficiently prevented, but also the myocardial viability and the systolic functions can be substantially improved, while the therapeutic myocardial angiogenesis, the myocardial capillary density and potential myogenesis in the pig model or patients can be enhanced. Furthermore, the biologically compatible peptide hydrogel in the infarcted area can be used to fasten and retain autologous peripheral blood stem cells (PBSCs) carried by blood flow through the infarcted area of myocardium tissue or the in situ endothelial or stem cells of an injured heart of a patient or a animal model for therapeutic angiogenesis in the heart after the pharmaceutical composition comprising the autologous stem cells (such as the autologous bone marrow mononuclear cells or the autologous umbilical cord blood stem cells) is administered, so as to be also advantageous to secondarily prevent heart failure after myocardial infarction and increase the myocardial angiogenesis, the myocardial capillary density and potential myogenesis.

As described above, in comparison with the traditional sustained delivery of PDGF using self-assembling peptide nanofibers in which PDGF only can promote the growth of live cardiomyocyte tissue remaining in the border zone of the affected part and cannot promote the growth of dead cardiomyocyte tissue in the central zone of the affected part, the method for improving myocardial infarction by intramyocardial or transendocardial injection of peptide nanofibers of the present invention, as shown in Figs. 1 and 2, the intramyocardial or transendocardial injection of peptide nanofibers (with the autologous stem cells, such as bone marrow mononuclear cells) is carried out at the 40 delivery sites (or delivery sites with suitable distribution density) over the entire infarcted area of the infarcted myocardium of the heart, few live cardiomyocytes remaining in the border zone and almost dead cardiomyocytes in the central zone over the entire infarcted area can be physically supported by the biologically compatible peptide hydrogel formed by the self-assembling peptide nanofibers, while the biologically compatible peptide hydrogel can be used to fasten and retain autologous stem cells, such as autologous adult stem cells, autologous induced pluripotent or multipotent stem cells or autologous peripheral blood stem cells (PBSCs) carried by blood flowing through the infarcted area or in situ endothelial or stem cells. As a result, the pathological ventricular remodeling and the diastolic dysfunction can be efficiently prevented, while the myocardial viability and the systolic functions can be substantially improved. Furthermore, the autologous stem cells retained within the infarcted area can be increased and the retention time of the autologous stem cells for cell therapy can be elongated, so that the therapeutic myocardial angiogenesis, the myocardial capillary density and potential myogenesis can be enhanced. Therefore, a potential clinical therapy for cardiac injury using intramyocardial or potentially, transendocardial injection of peptide nanofibers with autologous stem cells can be carried out.

The present invention has been described with a preferred embodiment thereof and it is understood that many changes and modifications to the described embodiment can be carried out.

## Claims

1. A pharmaceutical composition for repairing, or replacing a damaged tissue, comprising:
a biologically compatible peptide hydrogel comprising at least one self-assembling peptide nanofiber, wherein the self-assembling peptide nanofibers are structurally compatible to each other; and
at least one cell selected from the group consisting of autologous stem cells, progenitor cells, committed cells, and mature somatic cells.

2. The pharmaceutical composition as claimed in claim 1, wherein the damaged tissue is included in an organ of an organ system, and the organ system is selected from the group consisting of a circulatory system, a digestive system, an endocrine system, an excretory system, an integumentary system, a lymphatic system, a muscular system, a nervous system, a reproductive system, a respiratory system, and a skeletal system.

3. The pharmaceutical composition as claimed in claim 1, wherein the damaged tissue is included in an organ, which is selected from the group consisting of heart, blood vessels, salivary glands, esophagus, stomach, liver, gallbladder, pancreas, intestines, rectum, anus, hypothalamus, pituitary or pituitary gland, pineal body or pineal gland, thyroid, parathyroid, adrenals, kidneys, ureters, bladder, urethra, skin, tonsils, thymus, spleen, skeletal muscles, smooth muscles, brain, spinal cord, peripheral nerves, ovaries, fallopian tubes, uterus, vagina, mammary glands, testes, vas deferens, seminal vesicles, prostate, penis, pharynx, larynx, trachea, bronchi, lungs, diaphragm, bones, cartilage, ligaments, tendons, eye, eyelid, nose, face, lip, jaw, breast, and ear.

4. The pharmaceutical composition as claimed in any one of claims 1-3, wherein the damaged tissue is caused by ischemia, inflammation, infection, trauma or any other reason leading to tissue loss, defect or dysfunction.

5. The pharmaceutical composition as claimed in any one of claims 1-4, wherein the composition is for improving myocardial infarction.

6. The pharmaceutical composition as claimed in any one of claims 1-5, wherein the self-assembling peptide nanofibers have alternating hydrophobic and hydrophilic amino acids.

7. The pharmaceutical composition as claimed in any one of claims 1-6, wherein the self-assembling peptide nanofibers have a length of 8 to 200 amino acids.

8. The pharmaceutical composition as claimed in any one of claims 1-7, wherein the self-assembling peptide nanofibers are selected from the group consisting of SEQ ID NO 1, SEQ ID NO 2, SEQ ID NO 3, SEQ ID NO 4, SEQ ID NO 5, SEQ ID NO 6, SEQ ID NO 7, SEQ ID NO 8, SEQ ID NO 9, SEQ ID NO 10, SEQ ID NO 11, SEQ ID NO 12, SEQ ID NO 13, SEQ ID NO 14, SEQ ID NO 15, SEQ ID NO 16, SEQ ID NO 17, SEQ ID NO 18, SEQ ID NO 19, SEQ ID NO 20, SEQ ID NO 21, SEQ ID NO 22, SEQ ID NO 23, SEQ ID NO 24, SEQ ID NO 25, SEQ ID NO 26, SEQ ID NO 27, SEQ ID NO 28, SEQ ID NO 29, SEQ ID NO 30, SEQ ID NO 31, SEQ ID NO 32, SEQ ID NO 33, SEQ ID NO 34, SEQ ID NO 35, SEQ ID NO 36, SEQ ID NO 37, SEQ ID NO 38, SEQ ID NO 39, SEQ ID NO 40, SEQ ID NO 41, SEQ ID NO 42, SEQ ID NO 43, SEQ ID NO 44, SEQ ID NO 45, SEQ ID NO 46, SEQ ID NO 47, SEQ ID NO 48, SEQ ID NO 49, SEQ ID NO 50, and SEQ ID NO 51.

9. The pharmaceutical composition as claimed in any one of claims 1-8, wherein the self-assembling peptide nanofibers have a concentration of 0.1 % to 10% by weight.

10. The pharmaceutical composition as claimed in any one of claims 1-9, wherein the biologically compatible peptide hydrogel is prepared by dissolving the self-assembling peptide nanofibers in a buffer and mixing, and the buffer comprises monovalent metal cation selected from the group consisting of lithium, sodium, potassium, and combination thereof.

11. The pharmaceutical composition as claimed in any one of claims 1-10, wherein the autologous stem cells have a concentration of 10⁵ to 10¹⁰ cells per ml biologically compatible peptide hydrogel.

12. The pharmaceutical composition as claimed in any one of claims 1-11, wherein the autologous stem cell is selected from the group consisting of autologous adult stem cells, autologous induced pluripotent stem cells (iPS), autologous somatic progenitor cells, autologous committed, and mature somatic cells.

13. The pharmaceutical composition as claimed in claim 12, wherein the autologous adult stem cells are selected from the group consisting of bone marrow mononuclear cells, umbilical cord blood or placental stem cells, peripheral blood stem cells, dental stem cells, amniotic fluid stem cells, adipose stem cells, and somatic stem cells isolated from tissues.

14. The pharmaceutical composition as claimed in claim 12, wherein the somatic stem cell differentiates into committed cells, which are selected from the group consisting of cardiomyocytes, vascular smooth muscle cells, endothelial cells, pacemaker cells, and cardiac fibroblasts, esophagus cells, smooth muscle cells, endothelial cells, peptic cells, parietal cells, hepatocytes, gallbladder cells, pancreas cells, intestine cells, hypothalamus cells, pituitary cells, pituitary cells, thyroid cells, and parathyroid cells, adrenals, renal cells, smooth muscle cells, bladder cell, epithelial cell, fibroblast, tonsil cells, thymus cells, spleen cells, skeletal muscle cells, smooth muscle cells, endothelial cells, brain cells, myeloid cells, nervous cells, ovary cells, uterus cells, vagina cells, testes cells, prostate cells, pulmonary cells, smooth muscle cells, endothelial cells, ciliated cells, osteoblast, osteoclast, cartilage cell, ligament cells, and tendon cells.

15. The pharmaceutical composition as claimed in any one of claims 1-14, wherein the pharmaceutical composition is formulated into an injection, a superficial coating, or a patch.

16. A pharmaceutical composition as defined in any one of claims 1-15 for improving myocardial infarction.
